# EUROPEAN PATENT SPECIFICATION

(11) **EP 2 252 240 B1**
(45) Date of publication and mention of the grant of the patent: **01.08.2012**
(21) Application number: 09702554.8
(22) Date of filing: 13.01.2009
(51) Int. Cl.: A61F 2/84

(54) **STENT DELIVERY DEVICE WITH LUER OR CLAMP-TYPE SUTURE RELEASE APPARATUS**
STENTABGABEVORRICHTUNG MIT NAHTFREIGABEVORRICHTUNG VOM LUER- ODER KLEMMENTYP
APPAREIL DE RELACHEMENT DE SUTURE DE TYPE CLAMP ET PROCEDE DE CHARGEMENT ET D'ACHEMINEMENT DE STENT

(30) Priority: 14.01.2008 US 20822 P
(43) Date of publication of application: 24.11.2010
(73) Proprietor: Boston Scientific Scimed, Inc., Maple Grove, MN 55311 (US)
(72) Inventor: LENNA, Gary, J., Holden MA 01520 (US); WOOD, Mark, D., Shrewsbury MA 01545 (US)
(74) Representative: Vossius & Partner
(86) International application number: PCT/US2009/030819
(87) International publication number: WO 2009/091718

(56) References cited:
- US-A- 5 749 921
- US-A1- 2007 270 932
- US-A1- 2007 270 937

## Description

### CROSS-REFERENCE TO RELATED APPLICATIONS

This application claims the benefit of U.S. Provisional Application No. 61/020,822, filed January 14, 2008.

### FIELD OF THE INVENTION

The present invention relates to stent delivery assemblies. More particularly, this invention relates to methods and systems for transferring a stent from a radially expanded state to a radially compressed state prior to surgical implantation.

### BACKGROUND OF THE INVENTION

An intraluminary prosthesis, for example a stent, is a medical device used in the treatment of diseased bodily lumens. A stent is generally a longitudinal tubular device formed of biocompatible material which is useful to open and support various lumens in the body. For example, stents may be used in the bodily vessel, such as in the coronary or peripheral vasculature, esophagus, trachea, bronchi colon, biliary tract, urinary tract, prostate, brain, as well as in a variety of other applications in the body.

A stent generally includes an open flexible configuration which allows the stent to be configured in a radially compressed state for intraluminary catheter implantation. Once properly positioned adjacent the damaged vessel, the stent is radially expanded so as to support and reinforce the vessel. Radial expansion of the stent may be accomplished by inflation of a balloon attached to the catheter or the stent may be of the self-expanding variety which will radially expand once deployed.

Although stent delivery systems are well-known in the art, the assembly of such delivery systems is often complicated. Additionally, contemporary endoscopy practitioners increasingly use polymeric or plastic self-expanding stents. Unlike most metallic self-expanding stents, the polymeric stents have a tendency to permanently deform or lose some of their ability to self-expand when stored in a compressed state for a prolonged period of time. Such stents are therefore generally loaded into the stent delivery system shortly before being implanted in a patient. Such loading, however, often involves numerous steps, requires the use of multiple components (e.g., tools and fixtures) that are not part of the stent delivery system and/or required the practitioner to finish the loading process by manually pushing the stent into the delivery system by hand. Loading a stent in this way is therefore often difficult, time-consuming and has the potential to damage the stent.

Accordingly, there is a need for simplified methods of on-site loading of a stent into stent delivery systems, while minimizing the risk of damaging the stent in the process.

A stent delivery system is disclosed in US 2007/0270937 A1.

### SUMMARY OF THE INVENTION

The present invention is directed to a method and system for delivering a self-expanding stent into a body lumen. In particular, the present invention relates to an assembly and a method for protecting, loading and delivering a stent in combination with a stent delivery catheter, as well as to overall stent delivery systems.

The invention is described in claims 1 and 11 with preferable embodiments in the dependent claims.

Objectives, features, and advantages of this invention will become apparent from the following detailed description of illustrative embodiments thereof, which is to be read in connection with the accompanying drawings.

### BRIEF DESCRIPTION OF THE DRAWINGS

FIG. 1 illustrates a perspective view of an embodiment of a stent transfer and delivery system in accordance with the subject invention.
FIG. 2 illustrates a plan view of an embodiment of a stent transfer member in cross-section in accordance with the subject invention.
FIG. 3 illustrates an enlarged plan view of a distal portion of the assembly shown in FIG. 1, in cross-section.
FIG. 4 illustrates a plan view of the stent of FIG. 3 further detailing suture loops and suture threads.
FIG. 5 illustrates in a cross-sectional view the stent of FIG. 4 including a covering or a graft.
FIG. 6 illustrates an enlarged plan view of a distal portion of the assembly shown in FIG. 1, after the stent has been loaded in accordance with the subject invention.
FIG. 7 illustrates an enlarged plan view of an embodiment of a distal portion of the distal subassembly in cross-section, in accordance with the subject invention.
FIGS. 8 and 9 illustrate perspective, left side, plan and right side views, respectively, of an embodiment of the proximal handle in accordance with the subject invention.
FIGS. 10 and 11 further illustrate the cap of proximal handle of FIGS. 8 and 9

### DETAILED DESCRIPTION OF THE INVENTION

The present invention relates to an assembly for transporting and deploying a stent, or other intraluminary member as described herein, in a bodily passageway. The assembly is suited for medical applications (particularly, endoscopic therapy) in the gastrointestinal tract, the biliary tract, the urinary tract, and the respiratory tract. In particular, a preferred embodiment of the present invention is directed to an assembly for transporting, loading and delivering a self-expanding esophageal stent. The system allows the clinician or user to easily load a stent into a delivery system with minimal effort and without damaging the stent. Further, the assembly in accordance with the present invention could also be used in the neurological system (e.g., in the brain) and in the cardiovascular system (e.g., in the heart). Reference to bodily passageways may be to passageways in any of the aforementioned tracts and systems or elsewhere in the body.

It should be noted that references herein to the term "distal" are to a direction away from an operator of the subject invention, while references to the term "proximal" are to a direction towards the operator of the subject invention. Accordingly, when the terms "distal" and "proximal" are used herein in the context of an assembly device that is being deployed within a body, such as a human body, by an operator, the term "distal" refers to a location within the body that is further within the body than a location that is "proximal" to the operator.

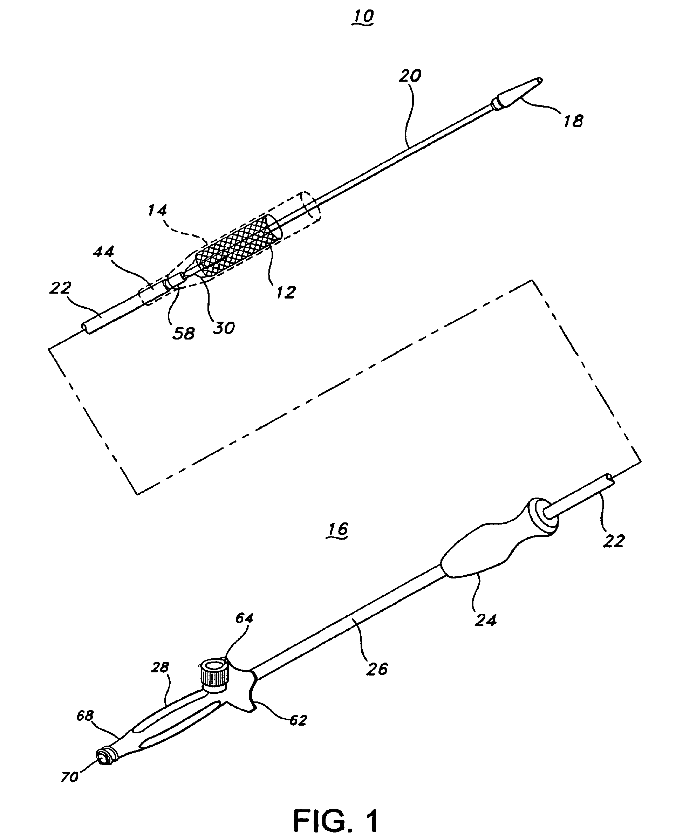

With reference to the drawings, FIG. 1 shows a perspective view of the stent delivery system **10** in accordance with a preferred embodiment of the subject invention. As seen in FIG. 1, a stent **12** is loaded within a stent transfer member **14** which is preferably attached to a stent delivery catheter subassembly **16.** The stent delivery catheter subassembly **16** preferably comprises a distal tip **18,** a distal inner member **20,** an outer tubular member **22,** a distal handle **24,** a proximal inner member **26,** and a proximal handle **28,** interrelated as shown. Further, as described in detail below, the stent delivery catheter subassembly **16** further includes a loading suture **30,** which is removeably coupled to the stent **12** and extends through the stent delivery catheter subassembly **16** to the proximal handle **28.**

While the present invention can be applied to the delivery of many intraluminary devices, it is particularly suited for delivering a self-expanding stent **12.** A preferred stent **12** should be capable of being radially compressed and longitudinally extended for implantation into a bodily lumen. The degree of elongation depends upon the structure and materials of the stent, and may be quite varied. The diameter of the stent also may become several times smaller as it elongates. It is preferred that the stent **12** be constructed to self-expand when released from a radially compressed state. Any stent that is capable of radial expansion is preferably used in accordance with the present invention. Further, the stent **12** may be repositionable, removable and/or reconstrainable, and/or may include multiple interconnected or non-interconnected stents. Various stent types and stent constructions may be employed in the invention, and the invention can be constructed to accommodate stents of various sizes and configurations.

One embodiment applies the method and system of the present invention to a braided stent **12.** As used herein the term braiding and its variants refer to the diagonal intersection of elongate filaments, such as elongate wires, so that each filament passes alternately over and under one or more of the other filaments, which is commonly referred to as an intersection repeat pattern. Useful braiding patterns include, but are not limited to, a diamond braid having a 1/1 intersection repeat pattern, a regular braid having a 2/2 intersection repeat pattern or a hercules braid having a 3/3 intersection repeat pattern. The passing of the filaments under and over one and the other results in slidable filament crossings that are not interlooped or otherwise mechanically engaged or constrained.

While the stent **12** can be formed of metals, plastics or other materials, it is preferred that a biocompatible construction is employed. Useful biocompatible materials include but are not limited to biocompatible metals, biocompatible alloys, biocompatible polymeric materials, including synthetic biocompatible polymeric materials and bioabsorbable or biodegradable polymeric materials, materials made from or derived from natural sources and combinations thereof. Useful biocompatible metals or alloys include, but not limited to, nitinol, stainless steel, cobalt-based alloy such as Elgiloy, platinum, gold, titanium, tantalum, niobium, polymeric materials and combinations thereof. Useful synthetic biocompatible polymeric materials include, but are not limited to, polyesters, including polyethylene terephthalate (PET) polyesters, polypropylenes, polyethylenes, polyurethanes, polyolefins, polyvinyls, polymethylacetates, polyamides, naphthalane dicarboxylene derivatives, silks and polytetrafluoroethylenes. The polymeric materials may further include a metallic, a glass, ceramic or carbon constituent or fiber. Useful and nonlimiting examples ofbioabsorbable or biodegradable polymeric materials include poly(L-lactide) (PLLA), poly(D,L-lactide) (PLA), poly(glycolide) (PGA), poly(L-lactide-co-D,L-lactide) (PLLA/PLA), poly(L-lactide-co-glycolide) (PLLA/PGA), poly(D,L-lactide-co-glycolide) (PLA/PGA), poly(glycolide-co-trimethylene carbonate) (PGA/PTMC), polydioxanone (PDS), Polycaprolactone (PCL), polyhydroxybutyrate (PHBT), poly(phosphazene) poly(D,L-lactide-co-caprolactone) PLA/PCL), poly(glycolide-co-caprolactone) (PGA/PCL), poly(phosphate ester) and the like. Further, the stent **12** may include materials made from or derived from natural sources, such as, but not limited to collagen, elastin, glycosaminoglycan, fibronectin and laminin, keratin, alginate, combinations thereof and the like.

Further, the stent **12** may be made from polymeric materials which may also include radiopaque materials, such as metallic-based powders or ceramic-based powders, particulates or pastes which may be incorporated into the polymeric material. For example, the radiopaque material may be blended with the polymer composition from which the polymeric filament is formed, and subsequently fashioned into the stent as described herein. Alternatively, the radiopaque material may be applied to the surface of the metal or polymer stent. Various radiopaque materials and their salts and derivatives may be used including, without limitation, bismuth, barium and its salts such as barium sulfate, tantalum, tungsten, gold, platinum and titanium, to name a few. Additional useful radiopaque materials may be found in U.S. Patent No. 6,626,936. Metallic complexes useful as radiopaque materials are also contemplated. The stent **12** may be selectively made radiopaque at desired areas along the stent **12** or made be fully radiopaque, depending on the desired end-product and application. Further, portions of the stent **12,** for example stent filaments, may have an inner core of tantalum, gold, platinum, iridium or combination of thereof and an outer member or layer of nitinol to provide a composite filament for improved radiocapicity or visibility. Alternatively, the stent **12** may also have improved external imaging under magnetic resonance imaging (MRI) and/or ultrasonic visualization techniques. MRI is produced by complex interactions of magnetic and radio frequency fields. Materials for enhancing MRI visibility include, but are not limited to, metal particles of gadolinium, iron, cobalt, nickel, dysprosium, dysprosium oxide, platinum, palladium, cobalt based alloys, iron based alloys, stainless steels, or other paramagnetic or ferromagnetic metals, gadolinium salts, gadolinium complexes, gadopentetate dimeglumine, compounds of copper, nickel, manganese, chromium, dysprosium and gadolinium. To enhance the visibility under ultrasonic visualization the stent **12** of the present invention may include ultrasound resonant material, such as but not limited to gold. Other features, which may be included with the stent **12** of the present invention, include radiopaque markers; surface modification for ultrasound, cell growth or therapeutic agent delivery; varying stiffness of the stent or stent components; varying geometry, such as tapering, flaring, bifurcation and the like; varying material; varying geometry of stent components, for example tapered stent filaments; and the like.

The stent transfer member **14** is preferably intended to protect a stent **12** or other similar inter-luminary device, before and during the time it is loaded into a delivery catheter lumen. Also, the stent transfer member **14** serves to safely radially compress the stent **12** for loading into a catheter lumen. In this way, the stent **12** can be loaded into the catheter lumen just prior to implantation in a patient's bodily passageway.

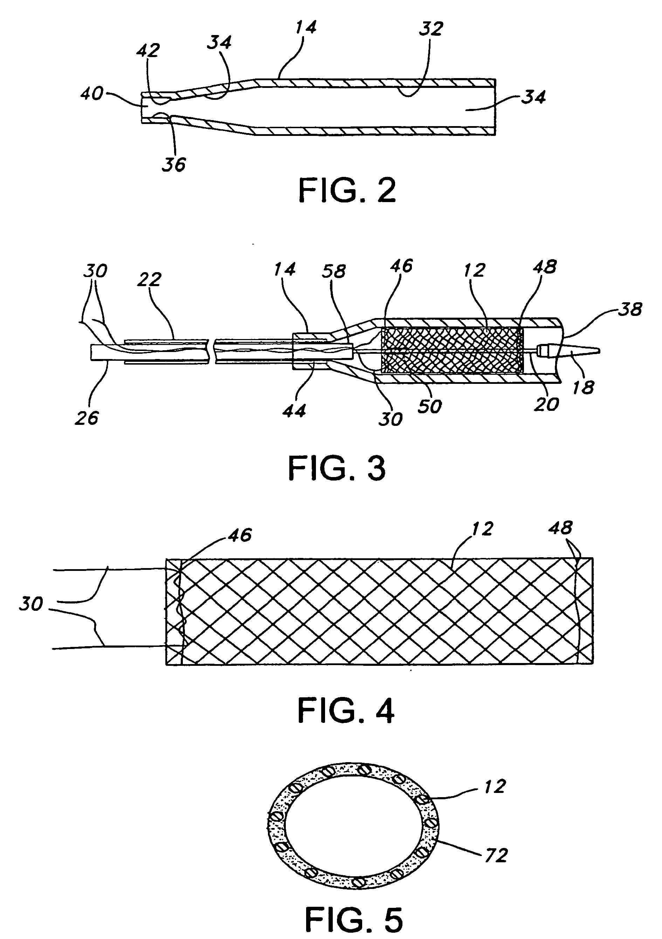

As shown in FIG. 2, an embodiment of the stent transfer member **14** is seen in cross-section separated from the overall delivery system **10.** The stent transfer member **14** preferably has a stent holding passage **32** whose inner diameter is preferably adapted to enclose a self-expanding stent in a fully radially expanded state. Alternatively, the stent holding passage **32** could have a somewhat smaller inner circumference in order to provide an element of frictional engagement with a stent **12** loaded therein. Further, although the stent holding passage **32** preferably encloses the entire length of the stent **12,** it could be longer or shorter. Thus, the holding passage **32** could be made to encircle only a portion of the stent **12.** The stent transfer member **14** also preferably includes a compression funnel passage **34** which serves to radially compress a stent **12** that passes from the stent holding passage to the more proximal catheter receiving passage **36.** The distal end **38** of the stent transfer member **14** is preferably open to allow unobstructed passage of the distal portions of the stent delivery catheter subassembly **16.** The proximal end **40** of the stent transfer member **14** is preferably adapted to engage with a distal end **44** of the outer tubular member **22.** Desirably, the proximal end **40** of the stent transfer member **14** has an inner cylindrical portion which acts as a catheter receiving passage **36** having a circumference that engages the outer circumference of the distal end **44** of the outer tubular member **22.** A transition step **42** preferably serves as a mating seat for the outer tubular member **22.** The transition step **42** is desirable to radially compress the stent **12** to the same or similar diameter as the inner lumen of the outer tubular member **22.**

The length or diameter of the stent transfer member **14** may be constructed to suit a particular application and/or stent. Also, the edges of the stent transfer member **14** could have a beveled profile. Further, the transfer member **14** could be constructed with one or more longitudinal slits or slots that can extend along the entire length or only a portion of the transfer member **14.** As a further alternative, the transfer member **14** could engage the outer tubular member **22** using other coupling techniques. Further still, as discussed above with regard to stents **12,** the transfer member **14** could be coated. Such coatings could reduce or enhance frictional engagement. Additionally, such coatings could further be designed to transfer or adhere to the stent **12** after it is removed from the transfer member **14.**

While the stent transfer member **14** is shown as a unitary member, it can alternatively be formed by separate elements or separable elements. In such a manner, the stent transfer member **14** could be made to split open or have a portion that can be removed to facilitate loading the stent **12** therein. For example, the stent transfer member **14** could be fractured, slit, split or hinged to provide the separate or separable elements. Such fractures, slits, splits, hinges, sites or detents for providing the separability function, either in part or in total, may be disposed along a longitudinal portion or axis of the stent transfer member **14,** along a circumferential portion or axis of the stent transfer member **14** and/or a combination thereof. Such an embodiment would, however, preferably provide some mechanism for holding the separate or separable elements together. Also, although a generally cylindrical outer structure is illustrated in FIG. 2, the stent transfer member **14** could have almost any shape to its outer surfaces. Whether to provide ergonomic features, a handle, engagement surfaces for tools, or simply ease of manufacture, it should be understood that the outer surfaces of the stent transfer member **14** could be altered from that shown. With regard to the inner surfaces **32, 34, 36** a cylindrical configuration is preferred, but alternative shapes are anticipated.

With reference to FIGS. 3 and 4, in accordance with the present invention a loading suture **30** or other suitable thread is preferably interlaced, braided, threaded, woven or otherwise disposed into a proximal end the stent **12.** The loading suture **30,** desirably a central or medial portion thereof, may be interlaced, braided, threaded, woven or otherwise disposed directly into or onto the wires, filaments or structure of the stent **12** itself. The central or medial portion of the loading suture **30** may be interlaced, braided, threaded, woven or otherwise disposed directly onto separate retrieval suture **46** that is part of the stent **12.** The loading suture **30** can be threaded through any number of loops of the proximal retrieval suture **46.** Further, the central or medial portion of the loading suture **30** may engage the wires, filaments or structure of the stent **12** and/or engage the retrieval suture **46.** The two ends of the loading suture **30** then preferably extend proximally from the stent **12** to the proximal end of the delivery system **10.**

Further, as depicted in FIGS. 3 and 4, retrieval sutures **48, 46** may located at either or both the distal and proximal ends of the stent **12.** The present invention, however, is not so limited, and the retrieval sutures **48, 46** may located at any point or points along the longitudinal and/or circumferential axis, individually or in combination, of the stent **12.** The retrieval sutures **48, 46** may be useful to a physician or practitioner after the stent is delivered into a body lumen. Such sutures **48, 46** desirably remain on the stent after it is implanted and allow the practitioner to reposition and/or remove the stent. Devices such as graspers or hooks can be used to pull on the retrieval sutures **48, 46.** When pulled, the retrieval suture **48, 46** is preferably adapted to constrict or radially contract the end of the stent in a purse string type movement. This constriction of an end of the stent **12** further makes it easier for the stent **12** to be pulled through an intraluminary passage of the stent transfer member **14** and into the stent delivery catheter subassembly **16.**

It should be noted that references herein to the term "suture" denotes a length of thread, thread-like member, cord, filament, wire or other similar structure. It should be understood that sutures as referred to herein can be made of a single material or composite materials. Accordingly, the terms "suture," "thread," "cord," "filament," and/or "wire" are used interchangeably herein.

As seen in FIG. 3, once the loading suture **30** is coupled to the stent **12** and fed through the delivery catheter subassembly **16** toward the proximal end of the assembly, the stent **12** is preferably loaded into the stent holding passage **32.** This can be done before or after the stent transfer member is mounted onto the distal end **44** of the outer tubular member **22.** The configuration shown in FIG. 3 maintains the stent **12** in a radially expanded state and can serve to protect the stent from the time of assembly until the stent is loaded into the lumen of outer tubular member **22.** Thus, the stent need not be compressed into a delivery catheter for an extended period, potentially causing permanent deformation.

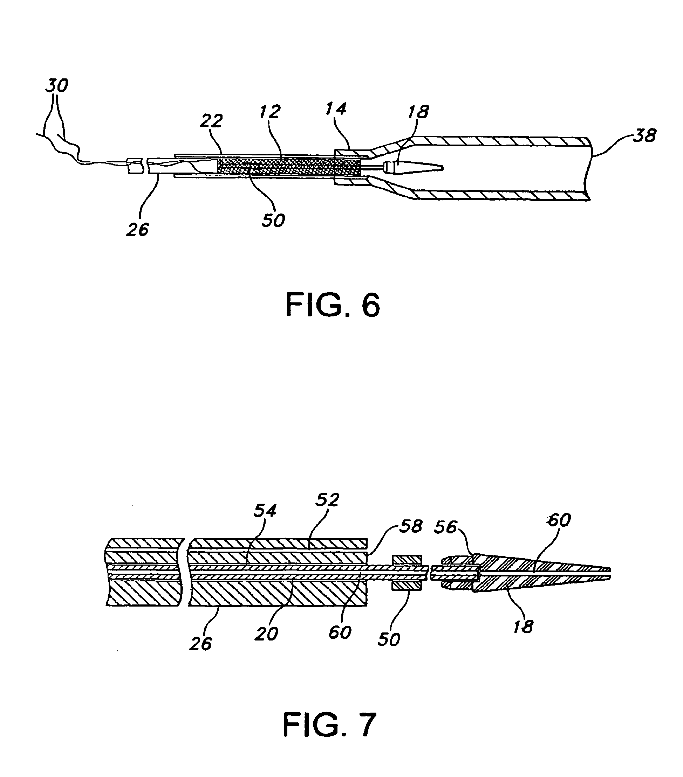

With reference to FIGS. 3 and 6, the distal inner member **20** and the proximal inner member **26** are preferably fixed to one another, functioning as a unitary member, along with distal tip **18.** These three inner members **18, 20, 26** are preferably coaxially configured within outer tubular member **22.** Also, as with a more traditional delivery catheter, the outer tubular member **22** is slidable axially relative to the three inner members **18, 20, 26.** Further, when the stent transfer member **14** is mounted onto the distal end **44,** it preferably slides axially in conjunction with the outer tubular member **22,** and thus also relative to inner members **18, 20, 26.** Thus, two handles are provided for manually sliding these elements relative to one another. The distal handle **24** controls the sliding movement of the outer tubular member **22,** along with, if attached, stent transfer member **14.** The proximal handle **28** likewise controls the sliding movement of the above mentioned inner members **18, 20, 26.** This relative sliding movement is used for both loading and deployment (delivery) of the stent **12.**

The inner members **20** and **26** and outer member **22** are preferably formed of a body compatible material. Desirably, the biocompatible material is a biocompatible polymer. Examples of suitable biocompatible polymers include, but are not limited to, polypropylene (PP), polytetrafluoroethylene (PTFE), polyethylene terephthalate (PET), high density polyethylene (HDPE) and the like. Materials for the members **20, 22, 26** may be the same or different. Additionally, the outer member **22** and the stent transfer member **14** could have coverings, films, coatings, and the like, desirably a polymeric covering, disposed over the inner surfaces to aid in the loading and/or deployment of the stent **12.**

The loading suture **30** preferably provides a link or coupling means between the stent **12** and the proximal end of the proximal inner member **26.** In a preferred embodiment, the loading suture **30** is removably secured to the proximal handle **28.** The desired purpose of securing the loading suture **30** is to limit the relative axial movement of the stent **12** away from the proximal inner member **26** and/or the proximal handle **28.** Thus, by moving the distal handle **24** away from the proximal handle **28,** the stent **12** is caused to be drawn through the compression funnel passage **34** and into an inner lumen of the outer tubular member **22,** as seen in FIG. 6. During this movement, the stent **12** is transferred from the stent holding passage **32** to the inner lumen of the outer tubular member **22.** Also, during this movement the stent **12** is preferably made to radially compress and engage onto the distal inner member **20,** or at least engage with the stent holder **50.** Thus, the configuration seen in FIG. 6 shows the stent **12** fully loaded within the inner lumen of the outer tubular member **22.** Alternatively, the distal end **44** of the outer tubular member **22** can include an inner bevel to aid in loading the stent **12.**

In one embodiment, the loading suture **30** can simply be threaded between the outer surface of the proximal inner member **26** and the inner surface of the outer tubular member **22.** Alternatively, the loading suture can be made to pass through auxiliary passage **52** in the proximal inner member **26,** seen in FIG. 7. The loading suture **30** can also be made to pass through the entire length of the proximal inner member **26,** the inside of the proximal handle **28** and out the proximal end of the proximal handle **28.** Alternatively, an additional opening can be made in the outer surface of the proximal inner member **26,** allowing the loading suture **30** to exit the auxiliary passage **52.** Such a loading suture **30** exit (not shown) can be disposed on the proximal inner member **26** at a location between the distal handle **24** and proximal handle **28.** Further, this alternative suture opening in the proximal inner member **26** can correspond with a stent release position discussed below.

During deployment or delivery of the stent **12,** as the two handles **24, 28** are drawn toward one another, there is a particular distance between them that corresponds with release of the stent **12.** As the proximal edge of the distal handle **24** slides along the surface of the proximal inner member **26,** a position on the surface of the proximal inner member **26** will correspond with the position that releases the stent **12** from the outer tubular member **22.** Thus, a marker for this release position can be provided on the surface of the proximal inner member **26.** Alternatively, the loading suture opening could be positioned to also function as this type of marker.

Also as depicted in FIG. 5, the materials of the stent **12** as well as the component filaments of the stent **12** can be further enhanced with coverings, films, coatings, and other materials and techniques. A covering, films or coating **72** may be in the form of a tubular structure, for example composed of polymeric material and/or silicone. The covering may also comprise any plastic or polymeric material, desirably a somewhat hard but flexible plastic or polymeric material. The covering may be transparent or translucent, desirably substantially or partially transparent. Furthermore, the covering may be constructed of any suitable biocompatible materials, such as, but not limited to, polymers and polymeric materials, including fillers such as metals, carbon fibers, glass fibers or ceramics. Useful covering materials include, but are not limited, polyethylene, polypropylene, polyvinyl chloride, polytetrafluoroethylene, including expanded polytetrafluoroethylene (ePTFE), fluorinated ethylene propylene, fluorinated ethylene propylene, polyvinyl acetate, polystyrene, poly(ethylene terephthalate), naphthalene dicarboxylate derivatives, such as polyethylene naphthalate, polybutylene naphthalate, polytrimethylene naphthalate and trimethylenediol naphthalate, polyurethane, polyurea, silicone rubbers, polyamides, polyimides, polycarbonates, polyaldehydes, polyether ether ketone, natural rubbers, polyester copolymers, styrene-butadiene copolymers, polyethers, such as fully or partially halogenated polyethers, and copolymers and combinations thereof. The coating or coatings **72** may be on the stent **12,** components of the stent **12,** and combinations thereof. The stent components, in part or in total, may be temporary, for example bioabsorbable, biodegradable, and the like, or may be permanent (i.e., not substantially bioabsorbable or biodegradable), for example the above-described biocompatible metals, alloys and polymers.

Further, the stent **12** and/or covering **72** may be treated with any of the following: anti-thrombogenic agents (such as heparin, heparin derivatives, urokinase, and PPack (dextrophenylalanine proline arginine chloromethylketone); anti-proliferative agents (such as enoxaprin, angiopeptin, or monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-miotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl keton, an RGD peptide-containing compound, heparin, antithrombin compounds, platelet receptor antagonists, antithrombin antibodies, anti-platelet receptor antibodies, aspirin, prostaglandin inhibitors, platelet inhibitors and tick antiplatelet peptides); vascular cell growth promotors (such as growth factor inhibitors, growth factor receptor antagonists, transcriptional activators, and translational promotors); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vascoactive mechanisms.

With reference to FIG. 7, the distal inner member **20** is preferably disposed and secured within an inner passage **54** in the proximal inner member **26.** Similarly, the stent holder **50** and the distal tip **18** are preferably fixed to the distal inner member **20.** These elements can be secured by frictional engagement or more permanent bonding. Alternatively, the distal inner member **20** and the proximal inner member **26** could be made as one unitary member. In this way, the distal subassembly seen in FIG. 7 will preferably move in unison. Also, the distal inner member **20** preferably extends through the entire length of the proximal inner member **26** and beyond it in the distal direction.

The distance between the distal end **58** of the proximal inner member **26** and the proximal end **56** of the distal tip **18** is preferably suited to accommodate the stent **12** in a radially compressed state, as seen in FIG. 6. It should be noted that as the stent **12** is radially compressed, it tends to axially expand. Thus, it is desired that the distance between the proximal inner member **26** and the distal tip **18** comfortably accommodate the axially expanded state of the stent **12.** The stent holder **50** is preferably provided to enhance the frictional engagement between the stent **12** and the distal inner member **20.** Once compressed onto the stent holder **50,** the stent **12** will slide axially in unison with the distal inner member **20,** unless the stent is radially released. Thus, as seen in FIG. 6, once compressed into the outer tubular member **22,** at least a portion of the stent **12** is preferably engaged with the stent holder **50.** Preferably, the stent holder **50** is made of a soft deformable or low durometer polymer that allows it to conform to the inner surface of the stent. For example, the stent holder **50** could be made from 2533 Pebax^{®} (ARKEMA, Courbevoie, France) a hardness 25, shore D, non-plasticized flexible polyamide, silicone, thermoplastic elastomer, such as but not limited to Dynaflex^{®} (GLS Corp., McHenry, IL), thermoplastic polyurethane elastomer and any other suitable material. However, it is understood that other suitable materials that function to enhance engagement with the stent could be used. Additionally, although the stent holder **50** is shown as an annular band, alternatively it could extend around only a portion or separate portions of the distal inner member **20.** In other words, the stent holder **50** may not have to completely encompass the distal inner member **20,** but may be only partially disposed around a circumferential portion thereof. Moreover, the stent holder **50** may have a pattern, such as a surface pattern of indentations and/or protrusions for facilitating securement of the stent **12.** In some embodiments, the stent holder **50** may have barbs, pins or protrusions which may engage the stent **12.** Further, with any of the embodiments, the device or system may include multiple stent holders **50,** either axially spaced apart or axially juxtaposed. The stent holder **50** may be short, i.e., having a longitudinal dimension encompassing only a portion of the longitudinal expanse of the stent **12,** or may be long, i.e., having a longitudinal expanse encompassing a substantial portion of the longitudinal expanse of the stent **12.**

Once the stent **12** is loaded as seen in FIG. 6, the stent transfer member **14** and/or the loading suture **30** can be removed from the assembly. Thereafter, the delivery catheter subassembly **16** that remains is preferably used to deliver the stent **12,** into a body lumen. In one alternative embodiment, the loading suture **30** need not be removed before delivery of the stent **12** into the patient. In this alternative embodiment, the loading suture **30** can be used to adjust the axial delivery position of the stent **12** in the proximal direction within the body lumen. In other words, the loading suture **30** is thus used to pull the stent **12** back to a more proximal location. Once positioned as desired, the loading suture **30** can be removed from the stent **12** as discussed below.

Removal of the stent transfer member **14** is preferably relatively simple. A frictional mounting between the applicable two elements **14, 44** is desirable, for easy removal. Alternatively, a screw-thread or other known means of engagement between the two elements could be provided.

With regard to the removal of the loading suture **30,** at least one end the loading suture **30** is preferably detached from the proximal handle **28** or the proximal inner member **26,** where it was secured. Then, by detaching and pulling the other end of the loading suture **30,** it is preferably pulled out of the stent **12** and the overall assembly. The loose weaving or braiding configuration between the loading suture **30** and the retrieval suture **46,** allows the suture **30** to be removed in this way. Even if the loading suture **30** were woven into the stent **12** itself, this removal technique could still suitably be used. Once the loading suture **30** has been removed from the stent **12** and the stent delivery catheter subassembly **16,** it can be set aside and/or discarded. In this way, with the stent transfer member **14** and loading suture **30** removed, the stent delivery catheter subassembly **16** is now loaded with a stent for delivery and can be inserted into a body lumen for delivery. The present invention, however, is not so limited to the above-described technique. For example, the loading suture **30** could be a biodegradable, bioabsorbable or dissolvable. In such as case the suture **30** may be left *in vivo.*

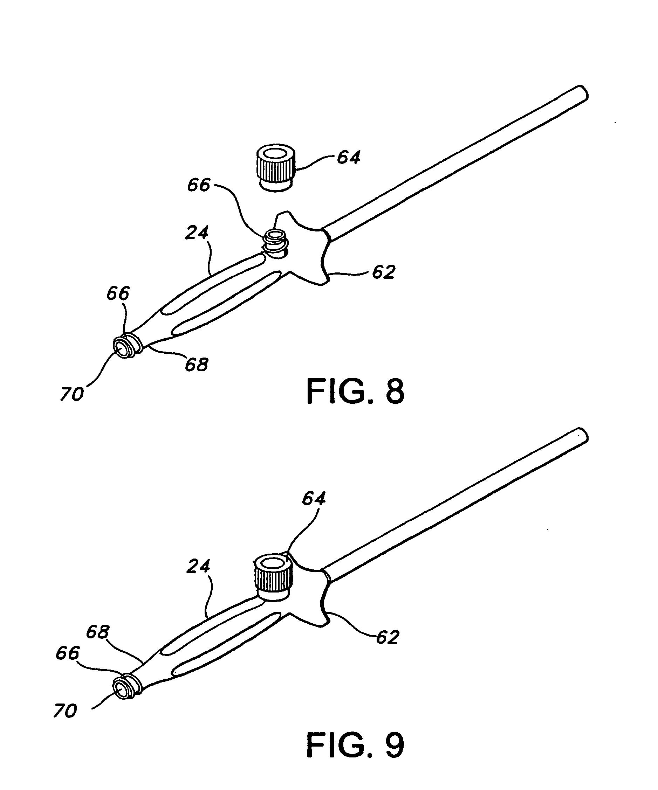

FIGS. 8 and 9 further illustrate the proximal handle **28,** which may be also referred to as a luer body. The proximal handle **28** shown in FIGS. 8 and 9 may include an auxiliary passage (not shown) that corresponds to auxiliary passage **52** in the proximal inner member **26.** Accordingly, the loading suture **30** may be fed through these auxiliary passages. The auxiliary passage of the proximal handle **28** may end either near or at either of the ports **66, 70.** Additionally, a luer flange **62** or other similar ergonomic feature may be provided on the distal end of the proximal handle **28.** The proximal handle 28 further includes a screw-on cap **64** as a suture lock. The screw-on cap **64** is preferably adapted to be mounted on a portion of the proximal handle **28,** for example onto a threaded cylindrical port **66** protruding from a lateral surface of the handle **28.** Alternatively, the same screw threading can be molded onto the proximal end **68** (depicted as port **70**) to receive the screw-on cap **64.** Additionally, the loading suture **30** can be fed through an inner passage in the port **66** or the flush passage **70,** and then secured by the screw-on cap **64.** As yet a further alternative, the mounting locations could engage the cap **64** with a snap lock design, rather than screw threads.

As part of an overall stent delivery system, it is preferred that an inner passage be provided for flushing fluids through the stent delivery catheter subassembly **16.** In this way, the proximal handle **28** is preferably provided with a flush passage **70** which traverses the length of the proximal handle **28.** This flush passage **70** is preferably open to and in communication with inner passage **52** in the distal inner member **20.** Further, this inner flow passage should preferably extend all the way through to an inner passage **60** in the distal tip **18.** In this way, an inner flush passage is provided from end to end in the overall assembly. Additionally, the proximal end of the proximal handle **28** can be molded to receive fluid flushing attachments or other surgical instruments.

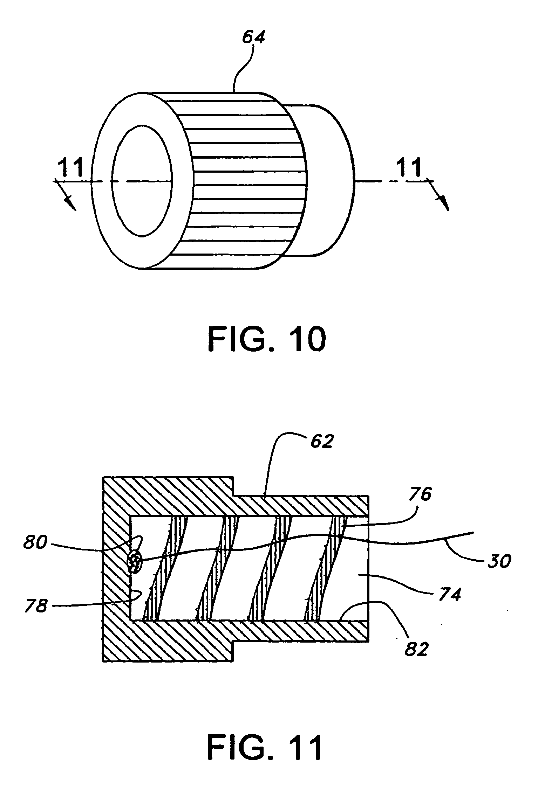

As depicted in FIGS. 9 and 10, one end of the loading suture **30** is permanently or releasably attached to one of the screw-on cap **64.** This can be done by injection molding, adhesives, heated bonding or other techniques. The one end of the suture thread **30** may be releasably or permanently secured by adhesive or bonding agent **80** at an inner top portion **78** of the cap **64.** Alternatively, the one end of the suture thread **30** may be releasably or permanently secured at an inner wall portion **82** of the cap **64** defined by the open bore **74** having grooves **76** engagable with the threaded portions cylindrical ports **66, 70.** It is desirable to allow the other end to be removable so that it can be pulled out of the stent **12** and the assembly **10,** as discussed above. As a further alternative, both ends of the loading suture **30** could be permanently or releasably secured, thus requiring the surgeon or user to simply cut them off prior to pulling the loading suture out. The ports **66** and/or **70** may secure the sutures in any number of manners. For example, the assembly **10** may include any suitable detent, including an enclosure, including but not limited to a cap, for releasably or permanently securing the thread **30** by, for example, a pinching mechanism, securing hooks or projections, tying techniques, and the like, or combinations thereof.

Additional features of useful stent delivery systems are further described in U.S. Patent Application Publication Nos. 2007-02790931 A1, 2007-02790932 A1 and 2007-02790937 A1.

Further, any of the components of the assembly **10** may have specific surface texturing and/or coatings to improve or affect loading, deployment and/or movement of the assembly **10** or its components.

While various embodiments of the present invention are specifically illustrated and/or described herein, it will be appreciated that modifications and variations of the present invention may be effected by those skilled in the art . Further, any of the embodiments or aspects of the invention as described in the claims or in the specification may be used with one and another without limitation.

## Claims

1. An assembly (10) for delivering a self-expanding stent (12) within a body lumen comprising:
(a) a self-expanding stent (12);
(b) a delivery catheter (16) for delivering the stent (12), the delivery catheter (16) comprising:
(i) an elongate flexible shaft (20) having a proximal end (26);
(ii) an elongate tube (22) adapted to enter the body lumen, the tube (22) moveably disposed over a portion of the shaft (20), the elongate tube (22) comprising a hollow cylindrical passage for containing the stent (12) in its radially compressed state;
(iii) a handle (28) secured to the proximal end (26) of the flexible shaft (20), the handle (28) having at least one open port (66) and at least one cap (64), releasably disposed thereat to cover the port (66);
(c) a transfer member (14) removeably engagable with a distal end of the delivery catheter (16), the transfer member (14) comprising a funnel-shaped passage (32, 34) for compressing a stent (12) from an at least partially radially expanded state to an at least partially radially compressed state when the stent (12) is moved through the passage (32, 34) of the transfer member (14) and into the hollow cylindrical passage (54) of the delivery catheter (16); and
(d) an elongate filament (30) for manipulating the stent (12) through the passage (32, 34) of the transfer member (14), the elongate filament (30) having first and second opposed ends and a medial portion disposed between the two opposed ends,
wherein the elongate filament (30) passes through a handle passage in at least a portion of the handle (28) and further wherein the medial portion is releasably secured to a proximal end of the stent (12) and the first and second opposed filament ends are accessible at the at least one open port (66) of the handle (28),
**characterized in that**
the at least one cap (64) comprises an inner top portion (78), and
the first opposed end of the filament (30) is secured to the inner-top portion (78) of the at least one cap (64).

2. The assembly (10) of claim 1, wherein the first opposed end of the filament (30) is secured to the inner top portion (78) of the at least one cap (64) by an adhesive or bonding agent (80).

3. The assembly (10) of claim 2, wherein the second opposed end of the filament (30) is secured to the inner top portion (78) of the at least one cap (64) by the adhesive or bonding agent (80).

4. The assembly (10) of claim 2,
wherein the at least one open port (66) further comprises threaded portions,
wherein the at least one cap (64) further comprises grooves (76) engagable with the threaded portions of the at least one open port (66), and
wherein the second opposed end of the filament (30) is releasably secured between the grooves (76) of the cap (64) and the threaded portions of the at least one open port (66).

5. The assembly (10) of claim 1, wherein the elongate flexible shaft (20) comprises a stent holder (50) at its distal end for restraining axial movement of the stent (12) when the stent (12) is in at least partially radially compressed state.

6. The assembly (10) of claim 1, wherein the elongate filament (30) comprises a suture, a thread, a cord, a wire and combinations thereof.

7. The assembly (10) of claim 1, wherein the elongate filament (30) passes through a supplemental passage (52) in at least a portion (26) of the flexible shaft (20).

8. The assembly (10) of claim 1, wherein the hollow cylindrical passage of the delivery catheter (16) is sized to surround the entire length of the stent (12).

9. The assembly (10) of claim 1, wherein the stent (12) is a braided stent.

10. The assembly (10) of claim 1, wherein the stent (12) comprises a biocompatible material, preferably a biocompatible polymeric material, even more preferably a bioabsorbable or biodegradable polymeric material.

11. A method for loading a self-expanding stent (12) into a stent delivery system (10) comprising:
(a) providing a self-expanding stent (12);
(b) providing a delivery catheter (16) for delivering the stent (12), the delivery catheter (16) comprising:
(i) an elongate flexible shaft (20) having a proximal end (26);
(ii) an elongate tube (22) adapted to enter the body lumen, the tube (22) moveably disposed over a portion of the shaft (20), the elongate tube (22) comprising a hollow cylindrical passage for containing the stent (12) in its radially compressed state;
(iii) a handle (28) secured to the proximal end (26) of the flexible shaft (20), the handle (28) having at least one open port (66) and at least one cap (64), releasably disposed thereat to cover the port (66);
(c) providing a transfer member (14) removeably engagable with a distal end of the delivery catheter (16), the transfer member (14) comprising a funnel-shaped passage (34) and a cylindrical passage (32), wherein the stent is disposed within the cylindrical passage (32) of the transfer member (14) in an at least partially radially expanded state;
(d) providing an elongate filament (30) for manipulating the stent through the passage (32, 34) of the transfer member (14), the elongate filament (30) having first and second opposed ends and a medial portion disposed between the two opposed ends, wherein the elongate filament (30) passes through a handle passage in at least a portion of the handle (28) and further wherein the medial portion is releasably secured to a proximal end of the stent (12) and the first and second opposed filament ends are accessible at the open port (66) of the handle (28);
(e) compressing the stent (12) from the at least partially radially expanded state to an at least partially radially compressed state by moving the stent (12) through the funnel-shaped passage (34) of the transfer member (14); and
(f) moving the stent (12) into the hollow cylindrical passage of the delivery catheter (16); **characterized in that**
the at least one cap (64) comprises an inner top portion (78), and at least the first opposed end of the filament (30) is secured, preferably by an adhesive or bonding agent (80), to the inner top portion (78) of the at least one cap (64), to limit relative axial movement of the stent (12) away from the handle (28).

12. The method of claim 11, wherein the elongate flexible shaft (20) comprises a stent holder (50) at its distal end for restraining axial movement of the stent (12) when the stent (12) is in at least partially radially compressed state.

13. The method of claim 11,
(i) wherein the second opposed end of the filament (30) is secured to the inner top portion (78) of the at least one cap (64) by the adhesive or bonding agent (80), or alternatively
(ii) wherein the at least one open port (66) further comprises threaded portions, where the at least one cap (64) further comprises grooves (76) engagable with the threaded portions of the at least one open port (66), and where the second opposed end of the filament (30) is releasably secured between the grooves (76) of the cap (64) and the threaded portions of the at least one open port (66).

14. The method of claim 11, further comprising:
removing the transfer member (14) from the distal end of the delivery catheter (16); and
moving the stent (12) outside of the delivery catheter (16),
wherein the step of removing the elongate filament (30) from the stent (12) is performed after the stent (12) is moved outside of the delivery catheter (16).

15. The method of claim 11, further comprising a step of repositioning the stent (12) comprising:
advancing the elongate flexible shaft (20) distally relative to the elongate tube (22) to move a portion of the stent (12) outside of the hollow cylindrical passage of the delivery catheter (16); and
manipulating the elongate filament (30) proximally in a direction toward the handle (28) to reposition at least part of the portion of the stent (12) back into the hollow cylindrical passage of the delivery catheter (16).

## Patentansprüche

1. Anordnung (10) zum Einführen eines selbstexpandierenden Stents (12) in ein Körperlumen, die aufweist:
(a) einen selbstexpandierenden Stent (12);
(b) einen Einführkatheter (16) zum Einführen des Stents (12), wobei der Einführkatheter (16) aufweist:
(i) einen länglichen flexiblen Schaft (20) mit einem proximalen Ende (26);
(ii) einen länglichen Schlauch (22), der geeignet ist, in das Körperlumen einzutreten, wobei der Schlauch (22) über einem Abschnitt des Schafts (20) beweglich angeordnet ist und der längliche Schlauch (22) einen hohlen zylindrischen Durchgang zum Aufnehmen des Stents (12) in dessen radial komprimiertem Zustand aufweist;
(iii) einen Griff (28), der am proximalen Ende (26) des flexiblen Schafts (20) befestigt ist, wobei der Griff (28) mindestens einen offenen Port (66) und mindestens eine Kappe (64) hat, die daran lösbar angeordnet ist, um den Port (66) abzudecken;
(c) ein Transferteil (14), das mit einem distalen Ende des Einführkatheters (16) entfernbar in Eingriff bringbar ist, wobei das Transferteil (14) einen trichterförmigen Durchgang (32, 34) zum Komprimieren eines Stents (12) aus einem mindestens teilweise radial expandierten Zustand in einen mindestens teilweise radial komprimierten Zustand aufweist, wenn der Stent (12) durch den Durchgang (32, 34) des Transferteils (14) und in den hohlen zylindrischen Durchgang (54) des Einführkatheters (16) bewegt wird; und
(d) ein längliches Filament (30) zum Manipulieren des Stents (12) durch den Durchgang (32, 34) des Transferteils (14), wobei das längliche Filament (30) ein erstes und ein zweites entgegengesetztes Ende sowie einen zwischen den beiden entgegengesetzten Enden angeordneten Mittelabschnitt hat,
wobei das längliche Filament (30) einen Griffdurchgang in mindestens einem Abschnitt des Griffs (28) durchläuft und wobei ferner der Mittelabschnitt an einem proximalen Ende des Stents (12) lösbar befestigt ist und das erste und zweite entgegengesetzte Filamentende am mindestens einen offenen Port (66) des Griffs (28) zugänglich sind, **dadurch gekennzeichnet, dass**
die mindestens eine Kappe (64) einen oberen Innenabschnitt (78) aufweist und
das erste entgegengesetzte Ende des Filaments (30) am oberen Innenabschnitt (78) der mindestens einen Kappe (64) befestigt ist.

2. Anordnung (10) nach Anspruch 1, wobei das erste entgegengesetzte Ende des Filaments (30) am oberen Innenabschnitt (78) der mindestens einen Kappe (64) durch einen Kleber oder Haftvermittler (80) befestigt ist.

3. Anordnung (10) nach Anspruch 2, wobei das zweite entgegengesetzte Ende des Filaments (30) am oberen Innenabschnitt (78) der mindestens einen Kappe (64) durch den Kleber oder Haftvermittler (80) befestigt ist.

4. Anordnung (10) nach Anspruch 2,
wobei der mindestens eine offene Port (66) ferner Gewindeabschnitte aufweist,
wobei die mindestens eine Kappe (64) ferner Nuten (76) aufweist, die mit den Gewindeabschnitten des mindestens einen offenen Ports (66) in Eingriff bringbar sind, und wobei das zweite entgegengesetzte Ende des Filaments (30) zwischen den Nuten (76) der Kappe (64) und den Gewindeabschnitten des mindestens einen offenen Ports (66) lösbar befestigt ist.

5. Anordnung (10) nach Anspruch 1, wobei der längliche flexible Schaft (20) einen Stenthalter (50) an seinem distalen Ende zum Einschränken von Axialbewegung des Stents (12) aufweist, wenn sich der Stent (12) im mindestens teilweise radial komprimiertem Zustand befindet.

6. Anordnung (10) nach Anspruch 1, wobei das längliche Filament (30) ein Nahtmaterial, einen Faden, eine Schnur, einen Draht und Kombinationen daraus aufweist.

7. Anordnung (10) nach Anspruch 1, wobei das längliche Filament (30) einen Zusatzdurchgang (52) in mindestens einem Abschnitt (26) des flexiblen Schafts (20) durchläuft.

8. Anordnung (10) nach Anspruch 1, wobei der hohle zylindrische Durchgang des Einführkatheters (16) so bemessen ist, dass er die Gesamtlänge des Stents (12) umgibt.

9. Anordnung (10) nach Anspruch 1, wobei der Stent (12) ein Flechtstent ist.

10. Anordnung (10) nach Anspruch 1, wobei der Stent (12) ein biokompatibles Material aufweist, vorzugsweise ein biokompatibles Polymermaterial, noch stärker bevorzugt ein biologisch absorbierbares oder biologisch abbaubares Polymermaterial.

11. Verfahren zum Laden eines selbstexpandierbaren Stents (12) in ein Stentabgabesystem (10), das aufweist:
(a) Bereitstellen eines selbstexpandierenden Stents (12);
(b) Bereitstellen eines Einführkatheters (16) zum Einführen des Stents (12), wobei der Einführkatheter (16) aufweist:
(i) einen länglichen flexiblen Schaft (20) mit einem proximalen Ende (26);
(ii) einen länglichen Schlauch (22), der geeignet ist, in das Körperlumen einzutreten, wobei der Schlauch (22) über einem Abschnitt des Schafts (20) beweglich angeordnet ist und der längliche Schlauch (22) einen hohlen zylindrischen Durchgang zum Aufnehmen des Stents (12) in dessen radial komprimierten Zustand aufweist;
(iii) einen Griff (28), der am proximalen Ende (26) des flexiblen Schafts (20) befestigt ist, wobei der Griff (28) mindestens einen offenen Port (66) und mindestens eine Kappe (64) hat, die daran lösbar angeordnet ist, um den Port (66) abzudecken;
(c) Bereitstellen eines Transferteils (14), das mit einem distalen Ende des Einführkatheters (16) entfernbar in Eingriff bringbar ist, wobei das Transferteil (14) einen trichterförmigen Durchgang (34) und einen zylindrischen Durchgang 32 aufweist, wobei der Stent im zylindrischen Durchgang (32) des Transferteils (14) in einem mindestens teilweise radial expandierten Zustand angeordnet wird;
(d) Bereitstellen eines länglichen Filaments (30) zum Manipulieren des Stents durch den Durchgang (32, 34) des Transferteils (14), wobei das längliche Filament (30) ein erstes und ein zweites entgegengesetztes Ende sowie einen zwischen den beiden entgegengesetzten Enden angeordneten Mittelabschnitt hat, wobei das längliche Filament (30) einen Griffdurchgang in mindestens einem Abschnitt des Griffs (28) durchläuft und wobei ferner der Mittelabschnitt an einem proximalen Ende des Stents (12) lösbar befestigt ist und das erste und zweite entgegengesetzte Filamentende am offenen Port (66) des Griffs (28) zugänglich sind;
(e) Komprimieren des Stents (12) aus dem mindestens teilweise radial expandierten Zustand in einen mindestens teilweise radial komprimierten Zustand durch Bewegen des Stents (12) durch den trichterförmigen Durchgang (34) des Transferteils (14); und
(f) Bewegen des Stents (12) in den hohlen zylindrischen Durchgang des Einführkatheters (16);
**dadurch gekennzeichnet, dass**
die mindestens eine Kappe (64) einen oberen Innenabschnitt (78) aufweist und mindestens das erste entgegengesetzte Ende des Filaments (30) am oberen Innenabschnitt (78) der mindestens einen Kappe (64) vorzugsweise durch einen Kleber oder Haftvermittler (80) befestigt ist, um relative Axialbewegung des Stens (12) weg vom Griff (28) zu begrenzen.

12. Verfahren nach Anspruch 11, wobei der längliche flexible Schaft (20) einen Stenthalter (50) an seinem distalen Ende zum Einschränken von Axialbewegung des Stents (12) aufweist, wenn sich der Stent (12) im mindestens teilweise radial komprimierten Zustand befindet.

13. Verfahren nach Anspruch 11,
(i) wobei das zweite entgegengesetzte Ende des Filaments (30) am oberen Innenabschnitt (78) der mindestens einen Kappe (64) durch den Kleber oder Haftvermittler (80) befestigt ist oder alternativ (ii) wobei der mindestens eine offene Port (66) ferner Gewindeabschnitte aufweist, wobei die mindestens eine Kappe (64) ferner Nuten (76) aufweist, die mit den Gewindeabschnitten des mindestens einen offenen Ports (66) in Eingriff bringbar sind, und wobei das zweite entgegengesetzte Ende des Filaments (30) zwischen den Nuten (76) der Kappe (64) und den Gewindeabschnitten des mindestens einen offenen Ports (66) lösbar befestigt ist.

14. Verfahren nach Anspruch 11, das ferner aufweist:
Entfernen des Transferteils (14) vom distalen Ende des Einführkatheters (16); und
Bewegen des Stents (12) außerhalb des Einführkatheters (16),
wobei der Schritt des Entfernens des länglichen Filaments (30) vom Stent (12) durchgeführt wird, nachdem der Stent (12) außerhalb des Einführkatheters (16) bewegt ist.

15. Verfahren nach Anspruch 11, ferner mit einem Schritt des Repositionierens des Stents (12), der aufweist:
distales Vorschieben des länglichen flexiblen Schafts (20) relativ zum länglichen Schlauch (22), um einen Abschnitt des Stents (12) außerhalb des hohlen zylindrischen Durchgangs des Einführkatheters (16) zu bewegen;
und
proximales Manipulieren des länglichen Filaments (30) in Richtung zum Griff (28), um mindestens einen Teil des Abschnitts des Stents (12) zurück in den hohlen zylindrischen Durchgang des Einführkatheters (16) zu repositionieren.

## Revendications

1. Ensemble (10) pour délivrer un stent auto-expansible (12) dans une lumière corporelle comprenant :
(a) un stent auto-expansible (12) ;
(b) un cathéter de délivrance (16) pour délivrer le stent (12), le cathéter de délivrance (16) comprenant :
(i) une tige flexible allongée (20) ayant une extrémité proximale (26) ;
(ii) un tube allongé (22) adapté pour pénétrer dans la lumière corporelle, le tube (22) disposé de manière mobile sur une partie de la tige (20), le tube allongé (22) comprenant un passage cylindrique creux pour contenir le stent (12) dans son état radialement compressé ;
(iii) une poignée (28) fixée à l'extrémité proximale (26) de la tige flexible (20), la poignée (28) ayant au moins un orifice ouvert (66) et au moins un capuchon (64), disposé de manière amovible sur celle-ci pour couvrir l'orifice (66) ;
(c) un membre de transfert (14) pouvant coopérer de manière amovible avec une extrémité distale du cathéter de délivrance (16), le membre de transfert (14) comprenant un passage en forme d'entonnoir (32, 34) pour compresser un stent (12) depuis un état au moins partiellement radialement expansé jusqu'à un état au moins partiellement radialement compressé quand le stent (12) est déplacé à travers le passage (32, 34) du membre de transfert (14) et dans le passage cylindrique creux (54) du cathéter de délivrance (16) ;
et
(d) un filament allongé (30) pour manipuler le stent (12) à travers le passage (32, 34) du membre de transfert (14), le filament allongé (30) ayant des première et seconde extrémités opposées et une partie médiane disposée entre les deux extrémités opposées,
où le filament allongé (30) passe à travers un passage de la poignée dans au moins une partie de la poignée (28) et où en outre la partie médiane est fixée de manière amovible à une extrémité proximale du stent (12) et les première et seconde extrémités opposées du filament sont accessibles au niveau du au moins un orifice ouvert (66) de la poignée (28),
**caractérisé en ce que**
le au moins un capuchon (64) comprend une partie supérieure interne (78), et
la première extrémité opposée du filament (30) est fixée à la partie supérieure interne (78) du au moins un capuchon (64).

2. Ensemble (10) selon la revendication 1, où la première extrémité opposée du filament (30) est fixée à la partie supérieure interne (78) du au moins un capuchon (64) par un agent adhésif ou liant (80).

3. Ensemble (10) selon la revendication 2, où la seconde extrémité opposée du filament (30) est fixée à la partie supérieure interne (78) du au moins un capuchon (64) par l'agent adhésif ou liant (80).

4. Ensemble (10) selon la revendication 2,
où le au moins un orifice ouvert (66) comprend en outre des parties filetées,
où le au moins un capuchon (64) comprend en outre des rainures (76) pouvant coopérer avec les parties filetées du au moins un orifice ouvert (66), et
où la seconde extrémité opposée du filament (30) est fixée de manière amovible entre les rainures (76) du capuchon (64) et les parties filetées du au moins un orifice ouvert (66).

5. Ensemble (10) selon la revendication 1, où la tige flexible allongée (20) comprend un dispositif de maintien de stent (50) à son extrémité distale pour limiter le mouvement axial du stent (12) quand le stent (12) est dans un état au moins partiellement radialement compressé.

6. Ensemble (10) selon la revendication 1, où le filament allongé (30) comprend une suture, un fil, un cordon, un fil métallique et leurs combinaisons.

7. Ensemble (10) selon la revendication 1, où le filament allongé (30) passe à travers un passage supplémentaire (52) dans au moins une partie (26) de la tige flexible (20).

8. Ensemble (10) selon la revendication 1, où le passage cylindrique creux du cathéter de délivrance (16) est dimensionné pour entourer toute la longueur du stent (12).

9. Ensemble (10) selon la revendication 1, où le stent (12) est un stent tressé.

10. Ensemble (10) selon la revendication 1, où le stent (12) comprend un matériau biocompatible, de préférence un matériau polymérique biocompatible, de préférence encore un matériau polymérique bioabsorbable ou biodégradable.

11. Procédé pour charger un stent auto-expansible (12) dans un système de délivrance de stent (10) comprenant :
(a) la fourniture d'un stent auto-expansible (12) ;
(b) la fourniture d'un cathéter de délivrance (16) pour délivrer le stent (12), le cathéter de délivrance (16) comprenant :
(i) une tige flexible allongée (20) ayant une extrémité proximale (26) ;
(ii) un tube allongé (22) adapté pour pénétrer dans la lumière corporelle, le tube (22) disposé de manière mobile sur une partie de la tige (20), le tube allongé (22) comprenant un passage cylindrique creux pour contenir le stent (12) dans son état radialement compressé ;
(iii) une poignée (28) fixée à l'extrémité proximale (26) de la tige flexible (20), la poignée (28) ayant au moins un orifice ouvert (66) et au moins un capuchon (64), disposé de manière amovible sur celle-ci pour couvrir l'orifice (66) ;
(c) la fourniture d'un membre de transfert (14) pouvant coopérer de manière amovible avec une extrémité distale du cathéter de délivrance (16), le membre de transfert (14) comprenant un passage en forme d'entonnoir (34) et un passage cylindrique (32), où le stent est disposé dans le passage cylindrique (32) du membre de transfert (14) dans un état au moins partiellement radialement expansé ;
(d) la fourniture d'un filament allongé (30) pour manipuler le stent à travers le passage (32, 34) du membre de transfert (14), le filament allongé (30) ayant des première et seconde extrémités opposées et une partie médiane disposée entre les deux extrémités opposées, où le filament allongé (30) passe à travers un passage de la poignée dans au moins une partie de la poignée (28) et où en outre la partie médiane est fixée de manière amovible à une extrémité proximale du stent (12) et les première et seconde extrémités opposées du filament sont accessibles au niveau de l'orifice ouvert (66) de la poignée (28) ;
(e) la compression du stent (12) depuis l'état au moins partiellement radialement expansé jusqu'à un état au moins partiellement radialement compressé par déplacement du stent (12) à travers le passage en forme d'entonnoir (34) du membre de transfert (14) ; et
(f) le déplacement du stent (12) dans le passage cylindrique creux du cathéter de délivrance (16) ;
**caractérisé en ce que**
le au moins un capuchon (64) comprend une partie supérieure interne (78), et au moins la première extrémité opposée du filament (30) et fixée, de préférence par un agent adhésif ou liant (80), à la partie supérieure interne (78) du au moins un capuchon (64) pour limiter le mouvement axial relatif du stent (12) à distance de la poignée (28).

12. Procédé selon la revendication 11, où la tige flexible allongée (20) comprend un dispositif de maintien de stent (50) à son extrémité distale pour limiter le mouvement axial du stent (12) quand le stent (12) est dans un état au moins partiellement radialement compressé.

13. Procédé selon la revendication 11
(i) où la seconde extrémité opposée du filament (30) est fixée à la partie supérieure interne (78) du au moins un capuchon (64) par l'agent adhésif ou liant (80), ou à titre d'alternative
(ii) où le au moins un orifice ouvert (66) comprend en outre des parties filetées, où le au moins un capuchon (64) comprend en outre des rainures (76) pouvant coopérer avec les parties filetées du au moins un orifice ouvert (66), et où la seconde extrémité opposée du filament (30) est fixée de manière amovible entre les rainures (76) du capuchon (64) et les parties filetées du au moins un orifice ouvert (66).

14. Procédé selon la revendication 11 comprenant en outre :
le retrait du membre de transfert (14) de l'extrémité distale du cathéter de délivrance (16) ; et
le déplacement du stent (12) à l'extérieur du cathéter de délivrance (16),
où l'étape de retrait du filament allongé (30) du stent (12) est accomplie après que le stent (12) soit déplacé à l'extérieur du cathéter de délivrance (16).

15. Procédé selon la revendication 11, comprenant en outre une étape de repositionnement du stent (12) comprenant :
l'avancement de la tige flexible allongée (20) distalement par rapport au tube allongé (22) pour déplacer une partie du stent (12) à l'extérieur du passage cylindrique creux du cathéter de délivrance (16) ; et
la manipulation du filament allongé (30) de manière proximale en direction de la poignée (28) pour repositionner au moins une partie de la partie du stent (12) de nouveau dans le passage cylindrique creux du cathéter de délivrance (16).
